# EUROPEAN PATENT SPECIFICATION

(11) **EP 3 659 999 B1**
(45) Date of publication and mention of the grant of the patent: **27.04.2022**
(21) Application number: 19775048.2
(22) Date of filing: 29.03.2019
(51) Int. Cl.: C07C 319/20, C07C 321/14, C07C 323/12, C07C 335/32, C08G 18/24, C08G 18/38, C08G 18/76, G02B 1/04

(54) **METHOD FOR PRODUCING POLYTHIOL COMPOUND, METHOD FOR PRODUCING CURABLE COMPOSITION, AND METHOD FOR PRODUCING CURED MATERIAL**
VERFAHREN ZUR HERSTELLUNG EINER POLYTHIOLVERBINDUNG, VERFAHREN ZUR HERSTELLUNG EINER HÄRTBAREN ZUSAMMENSETZUNG UND VERFAHREN ZUR HERSTELLUNG EINES GEHÄRTETEN MATERIALS
PROCÉDÉ DE PRODUCTION D'UN COMPOSÉ POLYTHIOL, PROCÉDÉ DE PRODUCTION D'UNE COMPOSITION DURCISSABLE, ET PROCÉDÉ DE PRODUCTION D'UN MATÉRIAU DURCI

(30) Priority: 29.03.2018 JP 2018064791
(43) Date of publication of application: 03.06.2020
(73) Proprietor: Hoya Lens Thailand Ltd., Pathumthani 12130 (TH)
(72) Inventor: KAGEYAMA, Yukio, Tokyo 160-8347 (JP)
(74) Representative: Hoffmann Eitle
(86) International application number: PCT/JP2019/014000
(87) International publication number: WO 2019/189762

(56) References cited:
- WO-A1-2007/129449
- WO-A1-2007/129450
- WO-A1-2018/003059
- WO-A1-2018/003059
- IN-A- 2342 DE2 004
- JP-A- 2015 506 947
- KR-A- 20120 058 635
- US-A1- 2015 126 781

## Description

### Technical Field

The present invention relates to a method for producing a polythiol compound, a method for producing a curable composition, and a method for producing a cured product.

### Background Art

A polythiol compound is widely used as a synthetic raw material for obtaining various resins. For example, a polythiourethane-based resin can be synthesized by a curing reaction between a polyiso(thio)cyanate compound and a polythiol compound (for example, refer to paragraph 0004 of Patent Literature 1).

### Citation List

### Patent Literature

Patent Literature 1: JP 5319037 B1

### Summary of Invention

### Technical Problem

As described in Patent Literature 1, a polythiol compound can be synthesized through the hydrolysis of an isothiuronium salt (for example, refer to paragraph 0036 of

### Patent Literature 1).

However, as a result of intensive studies by the present inventor, it has become clear that in a conventional method for producing a polythiol compound, reduction in coloration of the polythiol compound to be produced is required.

An aspect of the present invention provides a method for producing a polythiol compound capable of producing a polythiol compound with reduced coloration.

### Solution to Problem

The present inventor has got new finding conventionally unknown, during the repetition of intensive studies, that ammonium thiocyanate contained in thiourea to be used in synthesis of a polythiol compound largely affects the coloration of the polythiol compound. As a result of further intensive studies based on this finding, the present inventor has found a method for producing a polythiol compound according to an aspect of the present invention.

That is, an aspect of the present invention relates to the following method for producing a polythiol compound.

A method for producing a polythiol compound, the method including:
reacting a halide represented by the following Formula (1) with an alkali metal compound selected from the group consisting of an alkali metal sulfide and an alkali metal hydroxide to obtain a polyol compound selected from the group consisting of a polyol compound represented by Formula (2) and a polyol compound represented by Formula (3) (hereinafter, also described as "Step 1"), 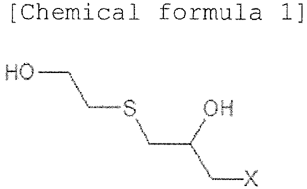
in Formula (1), X represents a halogen atom, 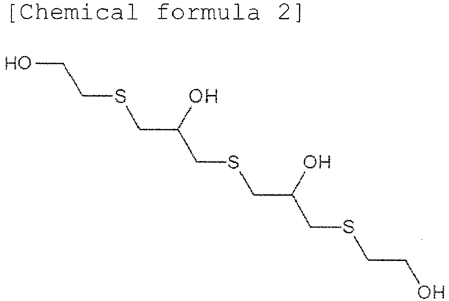 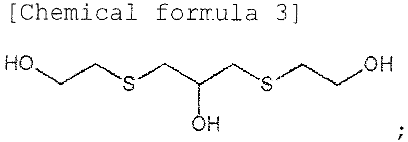
reacting the polyol compound with thiourea to obtain an isothiuronium salt (hereinafter, also described as "Step 2") ;
hydrolyzing the isothiuronium salt to obtain a polythiol salt (hereinafter, also described as "Step 3"); and
converting the polythiol salt into a polythiol to obtain one or more polythiol compounds selected from the group consisting of a polythiol compound represented by Formula (4), a polythiol compound represented by Formula (5), a polythiol compound represented by Formula (6), and a polythiol compound represented by Formula (7) (hereinafter, also described as "Step 4"), 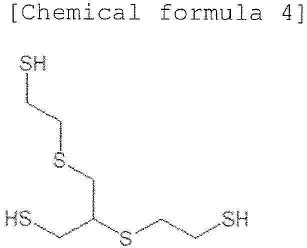 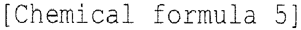 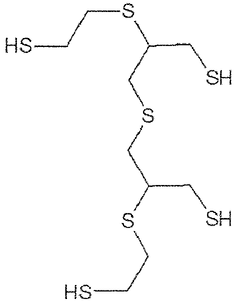 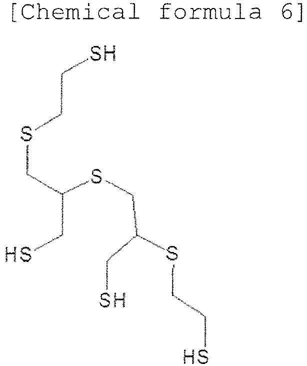 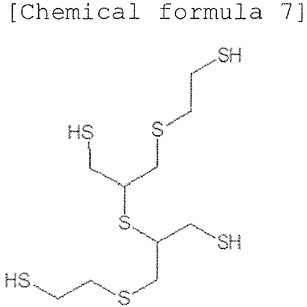
in which a content of ammonium thiocyanate in the thiourea is 0.05% by mass or less.

The present inventor has considered, during the repetition of intensive studies, that a complex formed by ammonium thiocyanate contained as an impurity in thiourea with a metal (for example, iron) contained as an impurity in an alkali metal compound used in reaction with the halide causes the coloration of a polythiol compound to be produced. On the contrary, in the above-described production method, thiourea in which a content of ammonium thiocyanate is 0.05% by mass or less is used as thiourea to be reacted with the polyol compound. According to this, it is speculated that the ability to suppress formation of the complex contributes to reduction in coloration of a polythiol compound to be produced. However, the above description is speculation, and the present invention is not limited to such speculation.

### Advantageous Effects of Invention

According to an aspect of the present invention, it is possible to reduce coloration of a polythiol compound which is useful as a synthetic raw material for various resins.

### Description of Embodiments

### [Method for producing polythiol compound]

Hereinafter, a method for producing a polythiol compound according to an aspect of the present invention will be described in more detail.

### <Step 1>

Step 1 is a step of reacting a halide represented by Formula (1) with an alkali metal compound selected from the group consisting of an alkali metal sulfide and an alkali metal hydroxide to obtain a polyol compound selected from the group consisting of a polyol compound represented by Formula (2) and a polyol compound represented by Formula (3) .

Formula (1) is as follows, and in Formula (1), X represents a halogen atom.

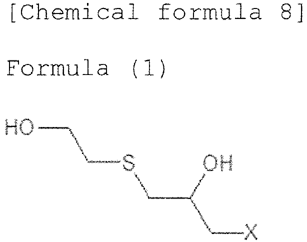

The halide represented by Formula (1) can be obtained, for example, by reacting 2-mercaptoethanol with epihalohydrin by the following reaction scheme example 1.

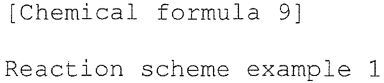

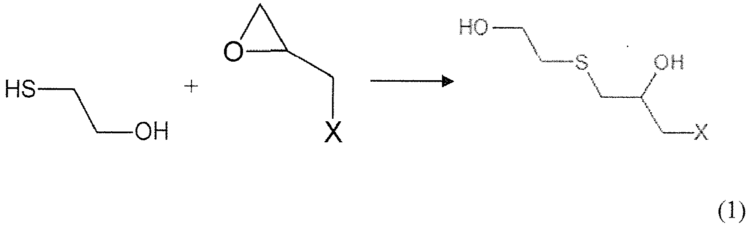

X in epihalohydrin of the reaction scheme example 1 and Formula (1) represents a halogen atom. The halogen atom is, for example, a fluorine atom, a chlorine atom, a bromine atom, and an iodine atom, and a chlorine atom and a bromine atom are preferable. For example, by using epichlorohydrin (X = chlorine atom) as epihalohydrin, a halide in which X in Formula (1) is a chlorine atom can be obtained. Further, by using epibromohydrin (X = bromine atom) as epihalohydrin, a halide in which X in Formula (1) is a bromine atom can be obtained.

The reaction between 2-mercaptoethanol and epihalohydrin is preferably performed in the presence of a catalyst. As the catalyst, various known catalysts can be used, and a tertiary amine can be used. As the tertiary amine, a tertiary alkyl amine is preferable. Specific preferred examples of the tertiary amine may include trimethylamine, triethylamine, tripropylamine, tributylamine, N,N-dimethylcyclohexylamine, and N,N-dicyclohexylmethylamine, and among these, triethylamine, tripropylamine, and tributylamine are more preferable and triethylamine and tributylamine are further preferable.

A reaction temperature in the reaction scheme example 1 and a reaction temperature in Step 1 can be set to, for example, about 0 to 60°C. A reaction time in the reaction scheme example 1 can be set to, for example, about 0.5 to 10 hours. The "reaction temperature" in the present invention and the present specification means the solution temperature of a reaction solution and is also described as an "internal temperature."

In an aspect, the synthesis reaction of the halide represented by Formula (1) by the reaction scheme example 1 can be carried out, for example, as follows. First, 2-mercaptoethanol is mixed to obtain a mixed solution. Here, it is preferable to mix a catalyst, and it is more preferable to mix a tertiary amine. Further, as necessary, a solvent (for example, alcohol or the like) may be added. Thereafter, epihalohydrin is added to the mixed solution. Epihalohydrin is preferably added dropwise to the mixed solution. A dropping time can be set to, for example, about 0.1 to 5 hours, but is not particularly limited. During the dropwise addition, the mixed solution may be stirred as necessary. Epihalohydrin can be reacted with 2-mercaptoethanol at a ratio of, for example, 0.5 to 3.0 mol, preferably 0.7 to 2.0 mol, and more preferably 0.9 to 1.1 mol with respect to 1.0 mol of 2-mercaptoethanol. The tertiary amine can be used in an amount of, for example, about 0.005 to 0.1 mol with respect to 1.0 mol of epihalohydrin. The mixed solution may be aged for about 0.5 to 10 hours as necessary after the addition of epihalohydrin. During the aging, the mixed solution may be left to stand still or may be stirred. As the tertiary amine, one or two or more tertiary amines can be used. The amount of the tertiary amine in the case of using two or more tertiary amines means the total content of these two or more tertiary amines. In the present invention and the present specification, components that can have different structures may be used singly or in combination of two or more kinds thereof unless otherwise specified. In a case where such two or more components are used, the content means the total content of the two or more components.

In Step 1, a halide represented by the above Formula (1) is reacted with an alkali metal compound selected from the group consisting of an alkali metal sulfide and an alkali metal hydroxide to obtain a polyol compound selected from the group consisting of a polyol compound represented by the following Formula (2) and a polyol compound represented by the following Formula (3).

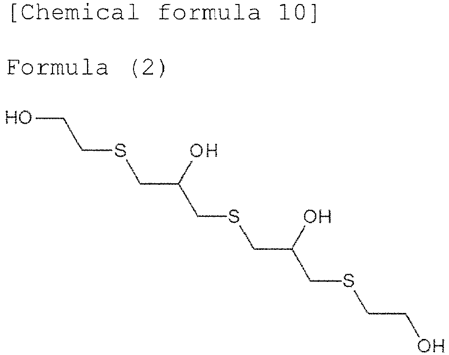

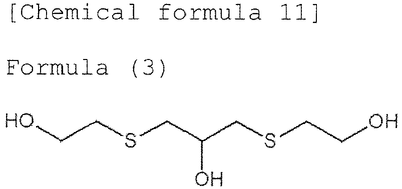

For example, when sodium sulfide is used as an example of the alkali metal compound, in Step 1, the polyol compound represented by Formula (2) can be obtained by the following reaction scheme example 2. Incidentally, numerical values described in the following reaction scheme example are based on a molar basis.

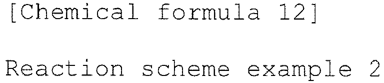

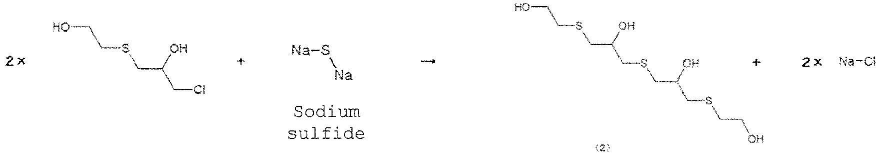

Further, when sodium hydroxide is used as an example of the alkali metal compound, in Step 1, the polyol compound represented by Formula (3) can be obtained by the following reaction scheme example 3.

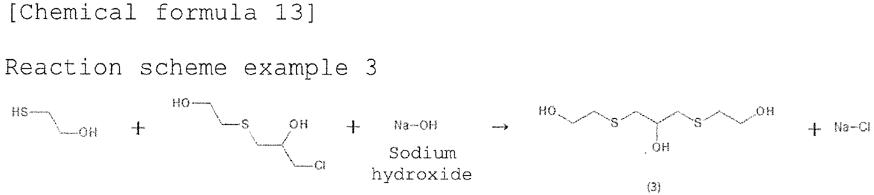

After the synthesis reaction of the halide represented by Formula (1) by the reaction scheme example 1 described above, a reaction solution containing the intended product obtained by the reaction (the halide represented by Formula (1)) may be used as it is in Step 1, or the reaction solution after the intended product is isolated by purifying the reaction solution after the reaction by a known method or the concentration of the reaction solution is increased can also be used in Step 1. Alternatively, the reaction solution after the synthesis reaction of the halide represented by Formula (1) by the reaction scheme example 1 described above can also be diluted with a solvent (for example, toluene or the like) and used in Step 1. The above points are also the same after the reactions of various steps described hereinafter. In an aspect using an alkali metal hydroxide as the alkali metal compound, as exemplified in the reaction scheme example 3, 2-mercaptoethanol is required to obtain the polyol compound represented by Formula (3). This 2-mercaptoethanol may be 2-mercaptoethanol which remains unreacted after the synthesis reaction of the halide represented by Formula (1) by the reaction scheme example 1 described above or may be newly added for Step 1. In Step 1, the amount of 2-mercaptoethanol used to obtain the polyol compound represented by Formula (3) can be set, for example, to 0.5 to 3.0 mol, preferably 0.7 to 2.0 mol, and more preferably 0.9 to 1.1 mol with respect to 1.0 mol of the halide represented by Formula (1).

In the reaction scheme examples 2 and 3 described above, an example in which the alkali metal atom contained in the alkali metal compound selected from the group consisting of alkali metal sulfide and alkali metal hydroxide is a sodium atom is shown. However, the alkali metal atom contained in the alkali metal compound may be another alkali metal atom such as a lithium atom or a potassium atom. In the reaction scheme example 2, the alkali metal compound is used at a ratio of, for example, 0.2 to 2.0 mol, preferably 0.3 to 1.2 mol, and more preferably 0.4 to 0.6 mol with respect to 1.0 mol of the halide represented by Formula (1), and in the reaction scheme example 3, the alkali metal compound is used at a ratio of, for example, 0.5 to 3.0 mol, preferably 0.7 to 2.0 mol, and more preferably 0.9 to 1.1 mol, whereby the alkali metal compound can be reacted with the halide represented by Formula (1). Incidentally, the alkali metal compound may be in the form of a hydrate. The amount of the hydrate means an amount containing hydrated water. The alkali metal compound may be used as it is for the reaction of Step 1 or may be used in the form of a solution such as an aqueous solution. In an aspect, the solution of the alkali metal compound can be added dropwise to a reaction solution containing the halide represented by Formula (1). A dropping time can be set to, for example, about 0.1 to 5 hours, but is not particularly limited. During the dropwise addition, the reaction solution may be stirred as necessary. Such a reaction solution may be aged for about 0.5 to 10 hours as necessary after the addition of the alkali metal compound. During the aging, the reaction solution may be left to stand still or may be stirred.

### <Step 2>

Next, Step 2 will be described.

Step 2 is a step of reacting the polyol compound obtained in Step 1 with thiourea to obtain an isothiuronium salt. Here, the present inventor has speculated that, when thiourea in which a content of ammonium thiocyanate is 0.05% by mass or less is used as thiourea, the formation of the complex described above is suppressed, and as a result, the coloration of the polythiol compound to be obtained by the production method can be reduced. From the viewpoint of further reducing the coloration of the polythiol compound, the content of ammonium thiocyanate in thiourea is preferably 0.04% by mass or less and more preferably 0.03% by mass or less. Further, the content of ammonium thiocyanate in thiourea can be set to, for example, 0.01% by mass or more, but from the viewpoint of coloration reduction, a lower content is preferable, and thus the content thereof may be the lower limit exemplified above. The content of ammonium thiocyanate in thiourea can be measured by using high performance liquid chromatography (HPLC).

The ammonium thiocyanate content in thiourea can be reduced by known various purification methods. As a preferable purification method as the method of reducing the ammonium thiocyanate content in thiourea, a recrystallization method can be exemplified. As a specific form of the recrystallization method, a method of precipitating (crystallizing) thiourea by using a good solvent with respect to ammonium thiocyanate can be exemplified. Here, as the good solvent with respect to ammonium thiocyanate, water, methanol, ethanol, acetone, and the like can be used.

Step 2 is a step of obtaining an isothiuronium salt as described above. The "isothiuronium salt" is a quaternary salt of isothiourea. The reaction between the polyol compound obtained in Step 1 and thiourea can be preferably performed in the presence of an acid. When hydrochloric acid is used as an example of the acid, for example, by reacting the polyol compound represented by Formula (2) with thiourea in the presence of an acid, an isothiuronium salt shown in the following reaction scheme example 4 can be obtained. Incidentally, in the reaction scheme example 4, an isothiuronium salt having the skeleton of a polythiol compound (5) is shown, but in this reaction, at least one isothiuronium salt selected from the group consisting of an isothiuronium salt having the skeleton of a polythiol compound (5), an isothiuronium salt having the skeleton of a polythiol compound (6), and an isothiuronium salt having the skeleton of a polythiol compound (7) can be obtained, and when the rearrangement reaction occurs, two or three of the isothiuronium salts can be obtained.

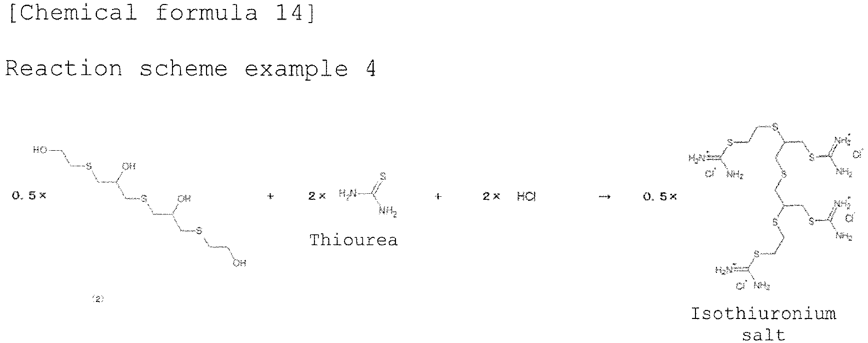

Further, for example, when the polyol compound represented by Formula (3) is reacted with thiourea in the presence of an acid, an isothiuronium salt having the skeleton of a polythiol compound (4) shown in the following reaction scheme example 5 can be obtained.

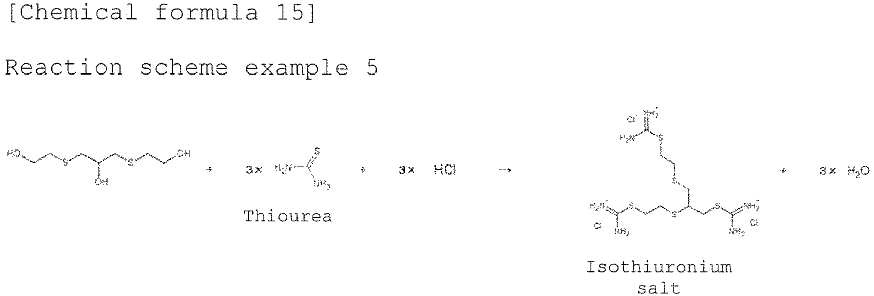

In the above reaction scheme examples 4 and 5, examples using hydrogen chloride (HCl) as an acid are shown, but the acid used in Step 3 is not limited to hydrogen chloride, and various inorganic acids and organic acids can be used. Examples of the inorganic acid may include hydrogen chloride and sulfuric acid, and examples of the organic acids may include formic acid. The form of addition of the acid is not particularly limited, but the acid can be added, for example, as an aqueous solution. The concentration of the acid in the aqueous solution is not particularly limited, but can be set to, for example, about 10 to 80% by mass. In Step 2, the acid can be used at a ratio of, for example, 2.0 to 12.0 mol, preferably 3.0 to 8.0 mol with respect to 1.0 mol of the polyol compound (in the reaction solution containing two or more polyol compounds, the total amount thereof is 1.0 mol), and thiourea can be used at a ratio of, for example, 3.0 to 6.0 mol, preferably 4.5 to 5.5 mol in the reaction scheme example 4, and for example, 2.0 to 5.0 mol, preferably 3.5 to 4.5 mol in the reaction scheme example 5. A reaction temperature in Step 2 can be set to, for example, 40°C to a reflux temperature, preferably about 90 to 120°C, and a reaction time can be set to, for example, about 1 to 24 hours.

### <Step 3>

Next, Step 3 will be described.

Step 3 is a step of hydrolyzing the isothiuronium salt obtained in Step 2 to obtain a polythiol salt. The polythiol salt obtained here is a salt having a structure in which a hydrogen atom of one or more thiol groups of three or four thiol groups (-SH) present in one molecule in the structure of the polythiol compound represented by Formula (4), Formula (5), Formula (6), or Formula (7) is substituted. In Step 3, two or more polythiol salts having different structures can also be obtained. The polythiol salt is preferably a polythiol alkali metal salt or a polythiol ammonium salt. The hydrolysis can be preferably performed in the presence of a base, and the type of the polythiol salt can be adjusted according to the type of the base used in the hydrolysis. As an example, an aspect of obtaining an alkali metal salt as the polythiol salt will be described below.

The polythiol alkali metal salt has a structure in which the isothiuronium salt obtained in Step 2 is hydrolyzed, as a result of which an alkali metal salt of a thiol group (-SM; M represents an alkali metal atom) is introduced into a molecular end. For example, a polythiol alkali metal salt (sodium salt) shown in the following reaction scheme example 6 can be obtained by hydrolyzing the isothiuronium salt having the skeleton of the polythiol compound (5) using sodium hydroxide as a base.

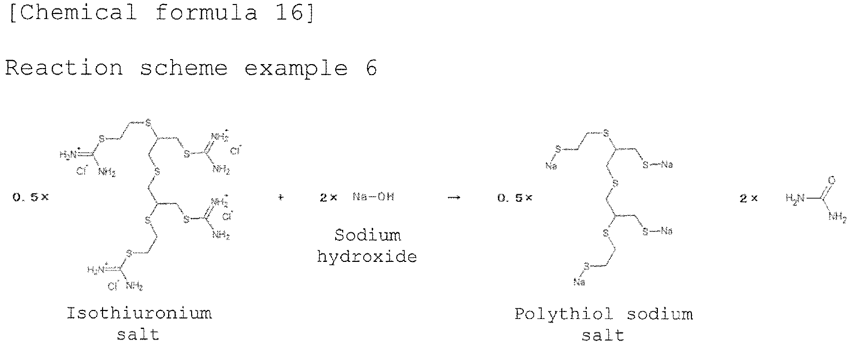

Further, a polythiol alkali metal salt (sodium salt) shown in the following reaction scheme example 7 can be obtained by hydrolyzing the isothiuronium salt having the skeleton of the polythiol compound (4) using sodium hydroxide as a base.

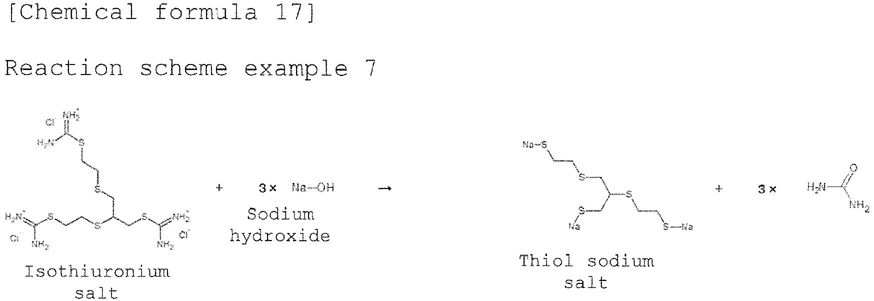

In the above reaction scheme examples 6 and 7, examples using sodium hydroxide as a base are shown, but the base used in Step 4 is not limited to sodium hydroxide, and various bases can be used. The base is preferably an inorganic base. Examples of the inorganic base may include sodium hydroxide, potassium hydroxide, and ammonia. The form of addition of the base is not particularly limited, and the base is preferably added as an aqueous solution. By adding the base as an aqueous solution, the isothiuronium salt can be hydrolyzed with water contained in the aqueous solution in the presence of the base. The concentration of the base in the aqueous solution is not particularly limited, but can be set to, for example, about 10 to 60% by mass. In the case of performing Step 2 in the presence of the acid, the base used in Step 3 can be used in a ratio of, for example, 1.0 to 4.0 mol, preferably 1.0 to 3.0 mol, and more preferably 1.2 to 2.0 mol with respect to 1.0 mol of the acid used in Step 2. Incidentally, an organic solvent can be added to the reaction solution containing the isothiuronium salt after the reaction in Step 2. Further, the organic solvent can be optionally added at any stage after the reaction in Step 2. The addition amount of the organic solvent can be set to, for example, about 0.2 to 3.0 times on a volume basis with respect to the amount of the reaction solution after the reaction in Step 2. Examples of the organic solvent may include toluene, xylene, chlorobenzene, and dichlorobenzene, and toluene is preferable. A reaction temperature in Step 3 can be set to, for example, about 10 to 80°C, and a reaction time can be set to, for example, about 1 to 10 hours.

### <Step 4>

Next, Step 4 will be described.

Step 4 is a step of converting the polythiol salt obtained in Step 3 into a polythiol. The conversion into a polythiol can be preferably performed by an acid. By converting the polythiol salt obtained in Step 3 into a polythiol, one or more polythiol compounds selected from the group consisting of a polythiol compound represented by Formula (4), a polythiol compound represented by Formula (5), a polythiol compound represented by Formula (6), and a polythiol compound represented by Formula (7) can be obtained. For example, a reaction scheme example 8 for converting the polythiol sodium salt shown in the reaction scheme example 6 into a polythiol using hydrogen chloride (HCl) as an acid to obtain the polythiol compound represented by Formula (5) is shown below.

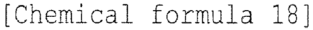

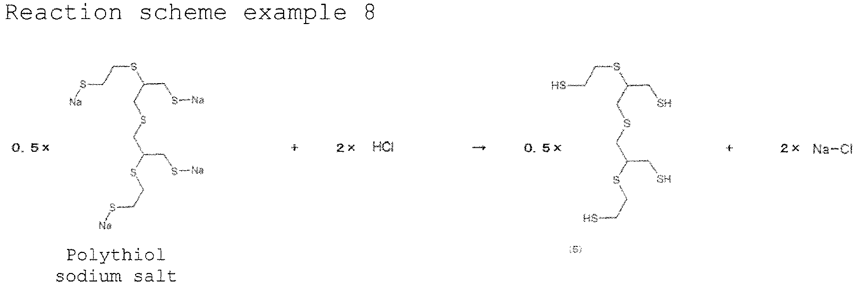

Further, a reaction scheme example 9 for converting the polythiol sodium salt shown in the reaction scheme example 6 into a polythiol using hydrogen chloride (HCl) as an acid to obtain the polythiol compound represented by Formula (4) is shown below.

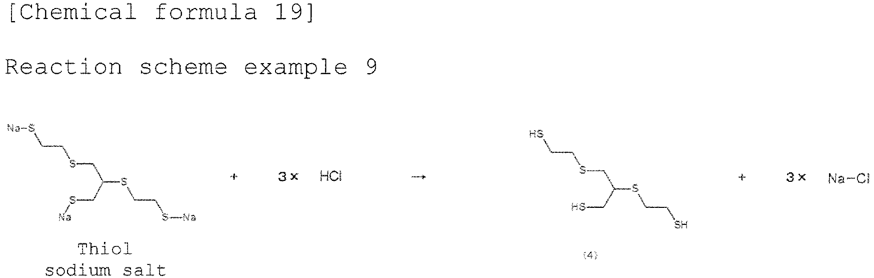

In the above reaction scheme examples 8 and 9, examples using hydrogen chloride as an acid are shown, but the acid used in Step 4 is not limited to hydrogen chloride, and various inorganic acids and organic acids can be used. The details of the acid used in Step 4 are as described above for the acid in Step 2. The conversion into a polythiol by an acid in Step 4 can be preferably performed by using an acid as an aqueous solution, and more preferably performed by acid washing. It is preferable to perform water washing after acid washing and it is more preferable to perform acid washing twice or more and perform water washing during the acid washing. The atmospheric temperature of an environment in which Step 4 is performed is not particularly limited. Step 4 can be performed under an environment of, for example, an atmosphere temperature of 10 to 60°C, preferably 20 to 45°C. Further, in the case of using an organic solvent in any of the steps, a step of distilling off the organic solvent from the reaction solution after Step 4 may be performed by a known method. Other than, post processes such as filtration and distillation can also be performed by known methods.

Each of the above-described steps can be carried out in the air and can also be carried out under an atmosphere other than the air, for example, under a nitrogen atmosphere.

According to the steps described above, one of the polythiol compound represented by Formula (4), the polythiol compound represented by Formula (5), the polythiol compound represented by Formula (6), and the polythiol compound represented by Formula (7), or a mixture of two or more thereof can be obtained. Incidentally, the degree of coloration of the polythiol compound can be evaluated by, for example, the b* value prescribed in JIS Z8781-4:2013. The b* value represents less coloration as the b* value is smaller. With the method for producing a polythiol compound according to an aspect of the present invention, for example, a polythiol compound having the b* value of 1.4 or less (for example, 0.5 to 1.4) can be obtained. In the case of obtaining a mixture of two or more polythiol compounds by Step 4, each of the polythiol compounds may be isolated by a known isolation method or the mixture may be used as a synthetic raw material for various resins. Each of the polythiol compounds represented by Formulae (4), (5), (6), and (7) and obtained by the method for producing a polythiol compound according to an aspect of the present invention is a polyfunctional polythiol compound having three or four thiol groups in one molecule. A cured product (polythiourethane-based resin) obtained by a curing reaction between such a polyfunctional polythiol compound and a polyiso(thio)cyanate compound can have various preferred physical properties as optical components such as a spectacle lens having a high refractive index, high heat resistance, and the like.

### [Curable composition and cured product]

An aspect of the present invention relates to a curable composition containing the polythiol compound obtained by the above-described production method and the polyiso(thio)cyanate compound.

Furthermore, an aspect of the present invention relates to a cured product obtained by curing the above-described curable composition.

Regarding the curable composition and the cured product, reference can be made to the descriptions relating to a method for producing a curable composition and a method for producing a cured product described below.

### [Method for producing curable composition]

An aspect of the present invention relates to a method for producing a curable composition, the method including:
producing a polythiol compound by the method for producing a polythiol compound according to the aspect of the present invention; and
mixing the produced polythiol compound with a polyiso(thio)cyanate compound to prepare a curable composition. By curing a curable composition obtained by the production method, a polythiourethane-based resin useful as a material of an optical component such as a spectacle lens can be obtained as a cured product. Hereinafter, the method for producing a curable composition will be described in more detail.

The details of the step of producing the polythiol compound are as described above for the method for producing a polythiol compound according to the aspect of the present invention described above. The curable composition can be prepared by mixing the polythiol compound produced in this way with the polyiso(thio)cyanate compound. In the present invention and the present specification, the "polyiso(thio)cyanate compound" is used to encompass a polyisocyanate compound and a polyisothiocyanate compound. Incidentally, isocyanate is sometimes referred to as isocyanate, and isothiocyanate is sometimes referred to as isothiocyanate. Further, the "isc(thio)cyanate group" is used to encompass an isocyanate group (-N=C=O) and an isothiocyanate group (-N=C=S). The "polyiso(thio)cyanate compound" is a polyfunctional compound having two or more iso(thio)cyanate groups in one molecule. By causing a curing reaction between a polythiol compound and a polyiso(thio)cyanate compound, a thiol group included in the polythiol compound reacts with an iso(thio)cyanate group included in the polyiso(thio)cyanate compound to obtain a reaction product having the following bond: 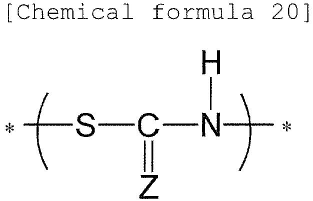 in a molecule. In the above, Z represents an oxygen atom or a sulfur atom. A reaction between the thiol group and the isocyanate group forms the bond in which X is an oxygen atom, and a reaction between the thiol group and the isothiocyanate group forms the bond in which X is a sulfur atom. In the present invention and the present specification, a reaction product (resin) containing a plurality of the bonds in one molecule is referred to as "polythiourethane-based resin".

As the polyiso(thio)cyanate compound, various polyiso(thio)cyanate compounds such as an aliphatic polyiso(thio)cyanate compound, an alicyclic polyiso(thio)cyanate compound, and an aromatic polyiso(thio)cyanate compound can be used. The number of iso(thio)cyanate groups contained in one molecule of the polyiso(thio)cyanate compound is 2 or more, preferably 2 to 4, and more preferably 2 or 3. Specific examples of the polyiso(thio)cyanate compound include various compounds exemplified as a polyiso(thio)cyanate compound in paragraph 0052 of JP 5319037 B1. Preferable examples of the polyiso(thio)cyanate compound include: an aliphatic polyisocyanate compound such as hexamethylene diisocyanate, 1,5-pentane diisocyanate, isophorone diisocyanate, bis(isocyanatomethyl) cyclohexane, dicyclohexylmethane diisocyanate, 2,5-bis(isocyanatomethyl)-bicyclo [2.2.1]heptane, 2,6-bis(isocyanatomethyl)-bicyclo [2.2.1]heptane, bis(4-isocyanatocyclohexyl) methane, 1,3-bis(isocyanatomethyl) cyclohexane, or 1,4-bis(isocyanatomethyl) cyclohexane; and an aromatic polyisocyanate compound such as bis(isocyanatomethyl) benzene, m-xylylene diisocyanate, p-xylylene diisocyanate, 1,3-diisocyanatobenzene, tolylene diisocyanate, 2,4-diisocyanatotoluene, 2,6-diisocyanatotoluene, or 4,4'-methylenebis(phenyl isocyanate). Furthermore, a halogen substitution product of the polyiso(thio)cyanate compound such as a chlorine substitution product thereof or a bromine substitution product thereof, an alkyl substitution product thereof, an alkoxy substitution product thereof, a nitro substitution product thereof, a prepolymer type modified product thereof with a polyhydric alcohol, a carbodiimide modified product thereof, a urea modified product thereof, a biuret modified product thereof, a dimerization or trimerization reaction product thereof, and the like can be used. These compounds may be used singly or in combination of two or more kinds thereof.

The curable composition can be prepared by mixing the polythiol compound with the polyiso(thio)cyanate compound. A mixing ratio between the polythiol compound and the polyiso(thio)cyanate compound in the curable composition is not particularly limited, but for example, as a molar ratio, a ratio of the thiol group contained in the polythiol compound/the iso(thio)cyanate group contained in the polyiso(thio)cyanate compound may be 0.5 to 3.0, preferably 0.6 to 2.0, and more preferably 0.8 to 1.3. The mixing ratio within the above range is preferable for obtaining a curable composition capable of providing a cured product having various excellent physical properties such as a high refractive index and high heat resistance. Further, in an aspect, the curable composition can contain, for example, 40% by mass or more (for example, 40 to 70% by mass) of the polythiol compound with respect to the total amount of 100% by mass of the curable composition.

At the time of preparing the curable composition, one or more components other than the polythiol compound and the polyiso(thio)cyanate compound may be mixed. Specific examples of such other components include a reaction catalyst for a curing reaction between a polythiol compound and a polyiso(thio)cyanate compound. For the other components that may be mixed, for example, refer to paragraphs 0055, 0057, and 0058 to 0064 of JP 5319037 B1. In addition, it is also possible to use one or more additives commercially available generally as additives for various resins such as a polythiourethane-based resin. The curable composition can be prepared by sequentially mixing the above-described various components at the same time or in any order. The preparation method is not particularly limited, and any known method for preparing a curable composition can be adopted without any limitation.

### [Method for producing cured product]

An aspect of the present invention relates to a method for producing a cured product, the method including:
producing a curable composition by the method for producing a curable composition according to the aspect of the present invention; and
curing the produced curable composition to obtain a cured product. Hereinafter, the method for producing a cured product will be described in more detail.

The details of the step of producing the curable composition are as described above for the method for producing a curable composition according to the aspect of the present invention described above. By curing a curable composition produced in this way, a polythiourethane-based resin useful as a material of an optical component such as a spectacle lens can be obtained as a cured product. According to the studies of the present inventor, it has become clear that by using the polythiol compound obtained by the method for producing a polythiol compound according to the aspect of the present invention as a synthetic raw material, a polythiourethane-based resin with reduced coloration is obtainable. The degree of coloration of the resin can be evaluated by, for example, a YI value prescribed in JIS K 7373:2006. The YI value represents less coloration as the YI value is smaller. By using the polythiol compound obtained by the method for producing a polythiol compound according to the aspect of the present invention as a synthetic raw material, a polythiourethane-based resin (cured product) having a YI value of, for example, 1.80 or less (for example, 0.50 to 1.80) can be obtained.

The curing reaction between the polythiol compound and the polyiso(thio)cyanate compound can be performed by various curing treatments capable of curing a curable composition. For example, cast polymerization is preferable for producing a cured product (also referred to as "plastic lens") having a lens shape. In cast polymerization, a curable composition is injected into a cavity of a molding die having two molds facing each other with a predetermined gap and a cavity formed by closing the gap, and the curable composition is subjected to polymerization (curing reaction) in the cavity to obtain a cured product. For the details of a molding die usable for cast polymerization, for example, reference can be made to paragraphs 0012 to 0014 and Fig. 1 of JP 2009-262480 A. Incidentally, the publication describes a molding die in which the gap between the two molds is closed with a gasket as a sealing member, but a tape can also be used as the sealing member.

In an aspect, the cast polymerization can be performed as follows. A curable composition is injected into a molding die cavity from an injection port provided on a side surface of the molding die. After the injection, by subjecting the curable composition to polymerization (curing reaction) preferably by heating, the curable composition is cured to obtain a cured product having an internal shape of the cavity transferred thereon. A polymerization condition is not particularly limited, and can be appropriately set depending on the composition of a curable composition or the like. As an example, a molding die having a curable composition injected into a cavity can be heated at a heating temperature of 20 to 150°C for about 1 to 72 hours, but the polymerization condition is not limited thereto. Incidentally, in the present invention and the present specification, the temperature such as a heating temperature for cast polymerization refers to a temperature of an atmosphere in which a molding die is placed. In addition, it is possible to raise the temperature at an arbitrary temperature rising rate during heating, and to lower the temperature (cooling) at an arbitrary temperature falling rate. After completion of the polymerization (curing reaction), the cured product inside the cavity is released from the molding die. The cured product can be released from the molding die by removing the upper and lower molds forming the cavity and a gasket or a tape in an arbitrary order as usually performed in cast polymerization. The cured product released from the molding die can be used preferably as a lens substrate of a spectacle lens. Note that the cured product used as a lens substrate of a spectacle lens can be usually subjected to a post-step such as annealing, a grinding step such as a rounding step, a polishing step, or a step of forming a coat layer such as a primer coat layer for improving impact resistance or a hard coat layer for improving surface hardness after releasing. Furthermore, various functional layers such as an antireflection layer and a water-repellent layer can be formed on the lens substrate. A known technique can be applied to any of these steps without any limitation. In this way, a spectacle lens in which a lens substrate is the cured product can be obtained. Furthermore, by mounting this spectacle lens in a frame, eyeglasses can be obtained.

### Examples

Next, the present invention will be described in more detail with Examples, but the present invention is not limited to aspects indicated by Examples. Operation and evaluation described below were performed in air at room temperature (about 20 to 25°C) unless otherwise specified. In addition, % and parts described below are on a mass basis unless otherwise specified.

Various physical properties in Examples and Comparative Examples were evaluated by the following methods.

### (1) Ammonium thiocyanate content in thiourea

The ammonium thiocyanate in thiourea used for Step 2 in Examples and Comparative Examples was quantitatively determined by high performance liquid chromatography (HPLC), and the ammonium thiocyanate content in thiourea was obtained from the quantitative determination result. The measurement conditions by HPLC were set as follows.
Measurement apparatus: Prominence HPLC system manufactured by SHIMADZU CORPORATION
Column: Shodex Asahipak NH2P-50G 4A
Length: 250 mm
Inner diameter: 4.6 mm
Film thickness: 5 µm
Mobile phase: Sodium phosphate buffer solution
Flow rate: 1.0 ml/min.
Column temperature: 40°C
Detector: ultraviolet-visible detector (210 nm)

The thiourea was quantitatively determined by HPLC described above and then provided to Step 2 within 1 hour. The content of ammonium thiocyanate is not changed, or even if the content of ammonium thiocyanate is changed, the amount of change is equal to or less than the detection limit by HPLC, during a period between the quantitative determination by HPLC and provision to Step 2.

### (2) Evaluation of coloration (b* value) of polythiol compound

The b* values of the polythiol compounds obtained in Examples and Comparative Examples were measured at an optical path length of 10 mm by using a spectrophotometer U-3500 manufactured by Hitachi, Ltd.

### (3) Evaluation of coloration (YI value) of cured product (polythiourethane-based resin)

The YI values of the cured products (0.00 D, center thickness: 1.8 mm) obtained in Examples and Comparative Examples were measured by using a spectral transmittance measuring apparatus DOT-3 manufactured by Murakami Color Research Laboratory.

It was confirmed by gel permeation chromatography (GPC), high performance liquid chromatography (HPLC), and mass spectrometry (MS) that intended polythiol compounds were obtained in the following Examples and Comparative Examples.

### [Pretreatment of thiourea (ammonium thiocyanate reduction treatment)]

When the ammonium thiocyanate content in commercially available thiourea was obtained by the method described in (1), the ammonium thiocyanate content was 0.50% by mass.

This commercially available thiourea was purified by a recrystallization method to obtain thiourea having a different ammonium thiocyanate content. In Example 1, thiourea obtained by drying a precipitate, which is precipitated by a recrystallization method after dissolving 500 g of the commercially available thiourea described above in 2000 g of pure water, under reduced pressure was used. The amount of the precipitate (thiourea) obtained after the drying under reduced pressure was 392 g, and the ammonium thiocyanate content was 0.01% by mass. Regarding other Examples and Comparative Examples, thiourea having a different ammonium thiocyanate content was prepared by appropriately changing the treatment conditions in the recrystallization method described above and then used in Step 2.

### [Example 1]

### <Synthesis of polythiol compound>

### (Synthesis of halide represented by Formula (1))

92.5 g (1.0 mol) of epichlorohydrin was added dropwise to a mixed solution of 78.1 g (1.0 mol) of 2-mercaptoethanol and 2.0 g of triethylamine over 1 hour while an internal temperature was maintained at 35 to 40°C, and the reaction solution was aged at an internal temperature of 40°C for 1 hour. According to this, a halide in which X in Formula (1) is a chlorine atom can be obtained. Incidentally, in this Example, other Examples, and Comparative Example, the reaction solution was aged while the reaction solution was stirred.

### (Step 1)

An aqueous solution prepared by dissolving 125.0 g (0.5 mol) of sodium sulfide nonahydrate in 100 g of pure water was added dropwise to the reaction solution after the aging over 1 hour while an internal temperature was maintained at 40 to 45°C, and the reaction solution was further aged at an internal temperature of 45°C for 1 hour.

### (Step 2 and Step 3)

303.8 g (3.0 mol) of 36% hydrochloric acid and 190.3 g (2.5 mol) of thiourea (ammonium thiocyanate content: 0.01% by mass) were added to the reaction solution after Step 1 described above, and the reaction solution was heated and stirred at an internal temperature of 110°C for 9 hours (Step 2). After the reaction solution was cooled to room temperature, 400 ml of toluene was added to the reaction solution, 600.4 g (4.5 mol) of a 30% sodium hydroxide aqueous solution was gradually added to the reaction solution, and the reaction solution was hydrolyzed at an internal temperature of 60°C for 4 hours (Step 3).

### (Step 4)

The reaction solution after Step 3 described above was left to stand still to separate the reaction solution into an aqueous layer and an organic layer, the organic layer was then taken out, and this organic layer was sequentially washed twice with 100 ml of 36% hydrochloric acid and 100 ml of water. Toluene in the organic layer after washing was distilled off with a rotary evaporator to obtain an intended polythiol compound.

In Example 1 described above, various reactions can be made to proceed as shown in the reaction scheme examples 1, 2, 4, 6, and 8. Incidentally, in the reaction scheme example 4, an isothiuronium salt having the skeleton of a polythiol compound represented by Formula (5) is shown, in the reaction scheme example 6, a polythiol alkali metal salt having the same skeleton is shown, and in the reaction scheme example 8, a polythiol compound represented by Formula (5) is shown, but in Step 2, as a result of the rearrangement reaction as described above, a mixture of an isothiuronium salt having the skeleton of the polythiol compound represented by Formula (5), an isothiuronium salt having the skeleton of the polythiol compound represented by Formula (6), and an isothiuronium salt having the skeleton of the polythiol compound represented by Formula (7) can be obtained, and as a result, in Step 4, a mixture of the polythiol compound represented by Formula (5), the polythiol compound represented by Formula (6), and the polythiol compound represented by Formula (7) can be obtained.

### <Production of plastic lens>

50.6 parts of xylylene diisocyanate, 0.01 parts of dimethyltin dichloride as a curing catalyst, 0.20 parts of acidic phosphate (JP-506H manufactured by Johoku Chemical Co., Ltd.) as a releasing agent, and 0.50 parts of ultraviolet absorber (Seesorb 701 manufactured by SHIPRO KASEI KAISHA, LTD.) were mixed and dissolved. Furthermore, 49.4 parts of the polythiol compound obtained above was added and mixed to obtain a mixed solution. This mixed solution was defoamed at 200 Pa for 1 hour, and then the resulting mixture was filtered with a polytetrafluoroethylene (PTFE) filter having a pore diameter of 5.0 µm. The mixed solution (curable composition) after filtration was injected into a lens molding die including a glass mold having a diameter of 75 mm and -4.00 D or 0.00 D and a tape. This molding die was put into an electric furnace, and the temperature was gradually raised from 15°C to 120°C over 20 hours and was kept for 2 hours for polymerization (curing reaction). After completion of the polymerization, the molding die was taken out from the electric furnace, and the resulting product was released from the molding die to obtain a cured product (plastic lens). The obtained plastic lens was further annealed for 3 hours in an annealing furnace having a furnace temperature of 120°C.

### [Examples 2 and 3 and Comparative Examples 1 and 2]

Synthesis of a polythiol compound and production of a cured product were performed in the same manner as in Example 1, except that thiourea having an ammonium thiocyanate content shown in the following Table 1 was used in Step 2.

**[Table 1]**

| | Ammonium thiocyanate content (%) in thiourea | b^{*} value of polythiol compound | YI value of plastic lens |
|---|---|---|---|
| Example 1 | 0.01 | 1.0 | 1. 67 |
| Example 2 | 0.02 | 1.2 | 1.73 |
| Example 3 | 0.05 | 1.3 | 1.77 |
| Comparative Example 1 | 0.06 | 1.6 | 2.08 |
| Comparative Example 2 | 0.10 | 2.1 | 2.69 |
| Comparative Example 4 | 0.10 | 2.0 | 2.62 |

### [Example 4]

### <Synthesis of polythiol compound>

### (Synthesis of halide represented by Formula (1))

46.3 g (0.5 mol) of epichlorohydrin was added dropwise to a mixed solution of 78.1 g (1.0 mol) of 2-mercaptoethanol and 2.0 g of tributylamine over 1 hour while an internal temperature was maintained at 35 to 40°C, and the reaction solution was aged at an internal temperature of 40°C for 1 hour.

### (Step 1)

44.4 g (0.5 mol) of a 45% sodium hydroxide aqueous solution was added dropwise to the reaction solution after the aging over 1 hour while an internal temperature was maintained at 40 to 45°C, and after the internal temperature was raised to 80°C, the reaction solution was aged for 1 hour.

### (Steps 2 and 3)

303.8 g (3.0 mol) of 36% hydrochloric acid and 114.2 g (1.5 mol) of thiourea (ammonium thiocyanate content: 0.01% by mass) were added to the reaction solution after Step 1 described above, and the reaction solution was heated and stirred at an internal temperature of 110°C for 2 hours (Step 2). After the reaction solution was cooled to room temperature, 200 ml of toluene was added to the reaction solution, 266.7 g (3.0 mol) of a 45% sodium hydroxide aqueous solution was gradually added to the reaction solution, and the reaction solution was hydrolyzed at 60°C for 4 hours (Step 3).

### (Step 4)

The reaction solution after Step 3 described above was left to stand still to separate the reaction solution into an aqueous layer and an organic layer, the organic layer was then taken out, and this organic layer was sequentially washed twice with 100 ml of 36% hydrochloric acid and 100 ml of water. Toluene in the organic layer after washing was distilled off with a rotary evaporator to obtain an intended polythiol compound.

In Example 4 described above, various reactions can be made to proceed as shown in the reaction scheme examples 1, 3, 5, 7, and 9.

### <Production of plastic lens>

52.0 parts of xylylene diisocyanate, 0.05 parts of dimethyltin dilaurate as a curing catalyst, 0.20 parts of acidic phosphate (JP-506H manufactured by Johoku Chemical Co., Ltd.) as a releasing agent, and 0.50 parts of ultraviolet absorber (Seesorb 701 manufactured by SHIPRO KASEI KAISHA, LTD.) were mixed and dissolved. Furthermore, 48.0 parts of the polythiol compound obtained above was added and mixed to obtain a mixed solution. This mixed solution was defoamed at 200 Pa for 1 hour, and then the resulting mixture was filtered with a polytetrafluoroethylene (PTFE) filter having a pore diameter of 5.0 µm. The mixed solution (curable composition) after filtration was injected into a lens molding die including a glass mold having a diameter of 75 mm and -4.00 D or 0.00 D and a tape. This molding die was put into an electric furnace, and the temperature was gradually raised from 30°C to 120°C over 20 hours and was kept for 2 hours for polymerization (curing reaction). After completion of the polymerization, the molding die was taken out from the electric furnace, and the resulting product was released from the molding die to obtain a cured product (plastic lens). The obtained plastic lens was further annealed for 3 hours in an annealing furnace having a furnace temperature of 120°C.

### [Examples 5 and 6 and Comparative Examples 3 and 4]

Synthesis of a polythiol compound and production of a cured product were performed in the same manner as in Example 4, except that thiourea having an ammonium thiocyanate content shown in the following Table 2 was used in Step 2.

**[Table 2]**

| | Ammonium thiocyanate content (%) in thiourea | b^{*} value of polythiol compound | YI value of plastic lens |
|---|---|---|---|
| Example 4 | 0.01 | 0.5 | 1.62 |
| Example 5 | 0.02 | 0.6 | 1.65 |
| Example 6 | 0.05 | 0.8 | 1.68 |
| Comparative Example 3 | 0.06 | 1.5 | 1.87 |
| Comparative Example 4 | 0.10 | 2.0 | 2.62 |

From the results shown in Table 1 and Table 2, it can be confirmed that by using thiourea in which a content of ammonium thiocyanate is 0.05% by mass or less in Step 2 described above, a polythiol compound with reduced coloration can be obtained, and by using such a polythiol compound, a plastic lens with reduced coloration can be obtained.

Finally, the above-described aspects will be summarized.

According to an aspect, there is provided a method for producing a polythiol compound, the method including: reacting a halide represented by Formula (1) with an alkali metal compound selected from the group consisting of an alkali metal sulfide and an alkali metal hydroxide to obtain a polyol compound selected from the group consisting of a polyol compound represented by Formula (2) and a polyol compound represented by Formula (3); reacting the polyol compound with thiourea to obtain an isothiuronium salt; hydrolyzing the isothiuronium salt to obtain a polythiol salt; and converting the polythiol salt into a polythiol to obtain one or more polythiol compounds selected from the group consisting of a polythiol compound represented by Formula (4), a polythiol compound represented by Formula (5), a polythiol compound represented by Formula (6), and a polythiol compound represented by Formula (7), in which a content of ammonium thiocyanate in the thiourea is 0.05% by mass or less.

According to the method for producing a polythiol compound, it is possible to provide a polythiol compound with reduced coloration.

In an aspect, the alkali metal compound may be an alkali metal compound selected from the group consisting of sodium sulfide and sodium hydroxide.

In an aspect, the polythiol salt may be a salt selected from the group consisting of a polythiol alkali metal salt and a polythiol ammonium salt.

According to another aspect, there is provided a method for producing a curable composition, the method including: producing a polythiol compound by the production method; and mixing the produced polythiol compound with a polyiso(thio)cyanate compound to prepare a curable composition.

According to the method for producing a curable composition, it is possible to obtain a curable composition capable of providing a cured product with reduced coloration.

According to still another aspect, there is provided a method for producing a cured product, the method including: producing a curable composition by the production method; and curing the produced curable composition to obtain a cured product.

According to the method for producing a cured product, it is possible to provide a cured product with reduced coloration.

In an aspect, the curing the produced curable composition to obtain a cured product can be performed by subjecting the curable composition to cast polymerization.

In an aspect, the cured product may be a spectacle lens substrate.

The embodiment disclosed herein is an example in every respect and should not be restrictively understood. The scope of the present invention is described not by the above description but by the claims.

An aspect of the present invention is useful in the field of producing various kinds of optical components such as a spectacle lens.

## Claims

1. A method for producing a polythiol compound, the method comprising:
reacting a halide represented by the following Formula (1) with an alkali metal compound selected from the group consisting of an alkali metal sulfide and an alkali metal hydroxide to obtain a polyol compound selected from the group consisting of a polyol compound represented by Formula (2) and a polyol compound represented by Formula (3), 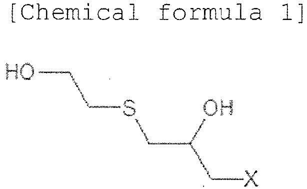 in Formula (1), X represents a halogen atom, 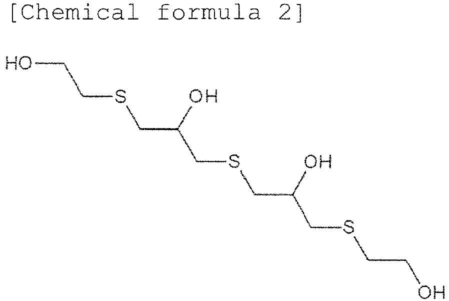 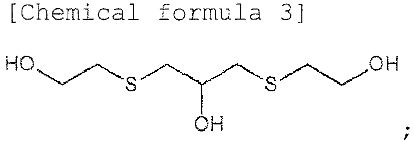 reacting the polyol compound with thiourea to obtain an isothiuronium salt;
hydrolyzing the isothiuronium salt to obtain a polythiol salt; and
converting the polythiol salt into a polythiol to obtain one or more polythiol compounds selected from the group consisting of a polythiol compound represented by Formula (4), a polythiol compound represented by Formula (5), a polythiol compound represented by Formula (6), and a polythiol compound represented by Formula (7), 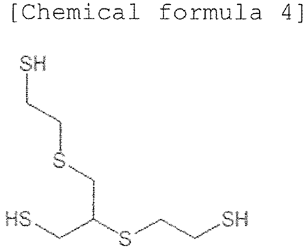 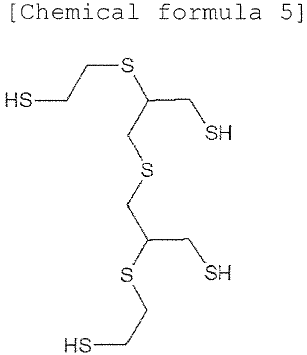 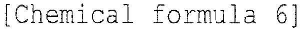 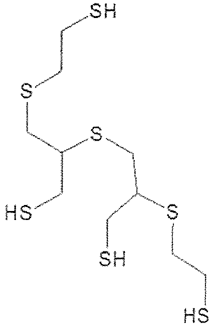 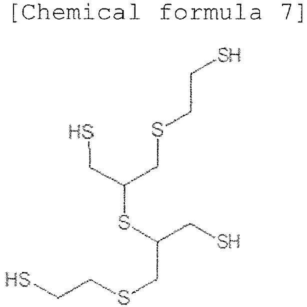
wherein a content of ammonium thiocyanate in the thiourea is 0.05% by mass or less.

2. The method for producing a polythiol compound according to claim 1, wherein the alkali metal compound is an alkali metal compound selected from the group consisting of sodium sulfide and sodium hydroxide.

3. The method for producing a polythiol compound according to claim 1 or 2, wherein the polythiol salt is a salt selected from the group consisting of a polythiol alkali metal salt and a polythiol ammonium salt.

4. A method for producing a curable composition, the method comprising:
producing a polythiol compound by the production method according to any one of claims 1 to 3; and
mixing the produced polythiol compound with a polyiso(thio)cyanate compound to prepare a curable composition.

5. A method for producing a cured product, the method comprising:
producing a curable composition by the production method according to claim 4; and
curing the produced curable composition to obtain a cured product.

6. The method for producing a cured product according to claim 5, wherein the curing the produced curable composition to obtain a cured product is performed by subjecting the curable composition to cast polymerization.

7. The method for producing a cured product according to claim 5 or 6, wherein the cured product is a spectacle lens substrate.

## Patentansprüche

1. Verfahren zur Herstellung einer Polythiolverbindung, wobei das Verfahren folgendes umfasst:
ein Halogenid mit der folgenden Formel (1) wird mit einer Alkalimetallverbindung, ausgewählt aus der Gruppe bestehend aus einem Alkalimetallsulfid und einem Alkalimetallhydroxid, so umgesetzt, dass eine Polyolverbindung, ausgewählt aus der Gruppe bestehend aus einer Polyolverbindung mit der Formel (2) und einer Polyolverbindung mit der Formel (3), erhalten wird, 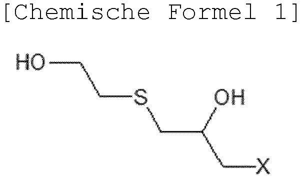
in der Formel (1) steht X für ein Halogenatom, 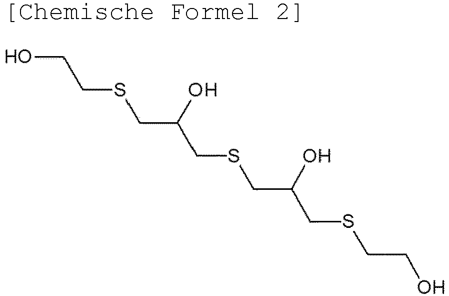 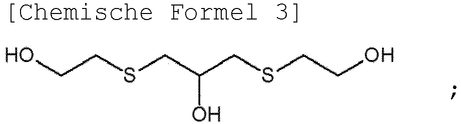
die Polyolverbindung wird mit Thioharnstoff so umgesetzt, dass ein Isothiouroniumsalz erhalten wird;
das Isothiouroniumsalz wird so hydrolysiert, dass ein Polythiolsalz erhalten wird; und
das Polythiolsalz wird in ein Polythiol so umgewandelt, dass ein oder mehr Polythiolverbindungen, ausgewählt aus der Gruppe bestehend aus einer Polythiolverbindung mit der Formel (4), einer Polythiolverbindung mit der Formel (5), einer Polythiolverbindung mit der Formel (6) und einer Polythiolverbindung mit der Formel (7), erhalten werden, 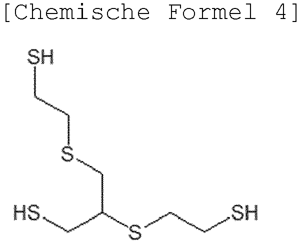 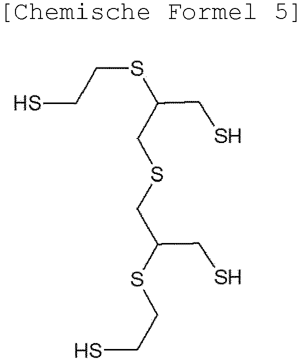 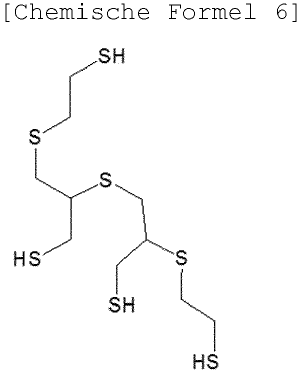 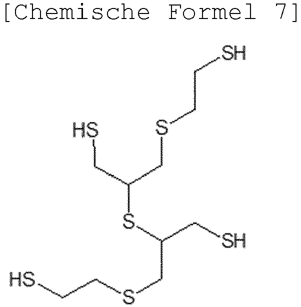
wobei ein Ammoniumthiocyanatgehalt in dem Thioharnstoff 0,05 Massen-% oder weniger beträgt.

2. Verfahren zur Herstellung einer Polythiolverbindung gemäß Anspruch 1, wobei die Alkalimetallverbindung eine Alkalimetallverbindung, ausgewählt aus Natriumsulfid und Natriumhydroxid, ist.

3. Verfahren zur Herstellung einer Polythiolverbindung gemäß Anspruch 1 oder 2, wobei das Polythiolsalz ein Salz, ausgewählt aus der Gruppe bestehend aus einem Polythiolalkalimetallsalz und einem Polythiolammoniumsalz, ist.

4. Verfahren zur Herstellung einer härtbaren Zusammensetzung, wobei das Verfahren folgendes umfasst:
eine Polythiolverbindung wird durch das Herstellungsverfahren gemäß mindestens einem der Ansprüche 1 bis 3 hergestellt; und
die hergestellte Polythiolverbindung wird mit einer Polyiso(thio)cyanatverbindung so gemischt, dass eine härtbare Zusammensetzung hergestellt wird.

5. Verfahren zur Herstellung eines gehärteten Produkts, wobei das Verfahren folgendes umfasst:
eine härtbare Zusammensetzung wird durch das Herstellungsverfahren gemäß Anspruch 4 hergestellt; und
die hergestellte härtbare Zusammensetzung wird so gehärtet, dass ein gehärtetes Produkt hergestellt wird.

6. Verfahren zur Herstellung eines gehärteten Produkts gemäß Anspruch 5, bei dem das Härten der hergestellten härtbaren Zusammensetzung zum Erhalten eines gehärteten Produkts durchgeführt wird, indem die härtbare Zusammensetzung einer Gusspolymerisation unterzogen wird.

7. Verfahren zur Herstellung eines gehärteten Produkts gemäß Anspruch 5 oder 6, bei dem das gehärtete Produkt ein Brillenglassubstrat ist.

## Revendications

1. Procédé de production d'un composé polythiol, le procédé comprenant :
la réaction d'un halogénure représenté par la formule (1) suivante avec un composé de métal alcalin sélectionné dans le groupe constitué par un sulfure de métal alcalin et un hydroxyde de métal alcalin pour obtenir un composé sélectionné dans le groupe constitué par un composé polyol représenté par la formule (2) et un composé polyol représenté par la formule (3), 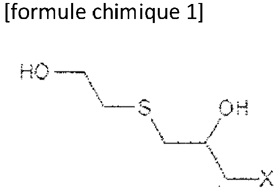
dans la formule (1), X représente un atome d'halogène, 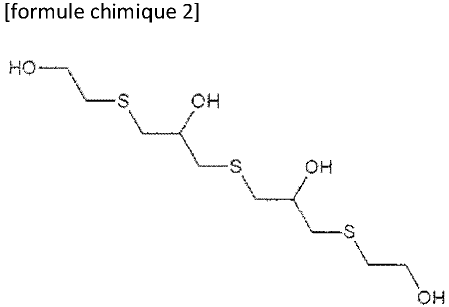 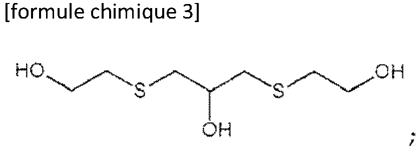
la réaction du composé polyol avec de la thiourée pour obtenir un sel d'isothironium ;
l'hydrolyse du sel d'isothironium pour obtenir un sel de polythiol ; et
la conversion du sel de polythiol en polythiol pour obtenir un ou plusieurs composés polythiol sélectionnés dans le groupe constitué par un composé polythiol représenté par la formule (4), un composé polythiol représenté par la formule (5), un composé polythiol représenté par la formule (6) et un composé polythiol représenté par la formule (7), 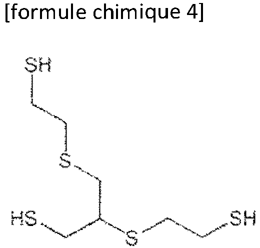 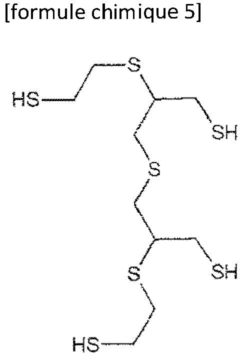 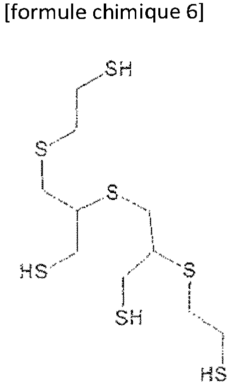 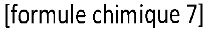 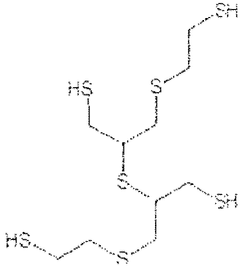
dans lequel une teneur en thiocyanate d'ammonium dans la thiourée est 0,05 % en masse ou moins.

2. Procédé de production d'un composé polythiol selon la revendication 1, dans lequel le composé métal alcalin est un composé métal alcalin sélectionné dans le groupe constitué par le sulfure de sodium et l'hydroxyde de sodium.

3. Procédé de production d'un composé polythiol selon la revendication 1 ou 2, dans lequel le sel de polythiol est un sel sélectionné dans le groupe constitué par un sel de métal alcalin de polythiol et un sel d'ammonium de polythiol.

4. Procédé de production d'une composition durcissable, le procédé comprenant :
la production d'un composé polythiol par le procédé de production selon l'une quelconque des revendications 1 à 3 ; et
le mélange du composé polythiol produit avec un composé polyiso(thio)cyanate pour préparer une composition durcissable.

5. Procédé de production d'un produit durci, le procédé comprenant :
la production d'une composition durcissable par le procédé de production selon la revendication 4 ; et
le durcissement de la composition durcissable produite pour obtenir un produit durci.

6. Procédé de production d'un produit durci selon la revendication 5, dans lequel le durcissement de la composition durcissable produite pour obtenir un produit durci est effectuée en soumettant la composition durcissable à une polymérisation par coulée.

7. Procédé de production d'un produit durci selon la revendication 5 ou 6, dans lequel le produit durci est un substrat de verre de lunettes.
